Europäisches Patentamt

European Patent Office

Office européen des brevets

(19)

(11) Veröffentlichungsnummer: **0 120 440**
**B1**

(12) **EUROPÄISCHE PATENTSCHRIFT**

(45) Veröffentlichungstag der Patentschrift: 08.08.90

(51) Int. Cl.⁵: **C 12 N 9/04, C 12 Q 1/32**

(21) Anmeldenummer: 84103042.2

(22) Anmeldetag: 20.03.84

(54) **NAD(P)-unabhängige Glycerindehydrogenase, Verfahren zu deren Herstellung sowie deren Verwendung zur Bestimmung von Glycerin und Triglyceriden.**

(30) Priorität: 25.03.83 DE 3311027

(43) Veröffentlichungstag der Anmeldung:
03.10.84 Patentblatt 84/40

(45) Bekanntmachung des Hinweises auf die
Patenterteilung:
08.08.90 Patentblatt 90/32

(84) Benannte Vertragsstaaten:
AT BE CH DE FR GB IT LI LU NL SE

(56) Entgegenhaltungen:
CHEMICAL ABSTRACTS, Band 46, Nr. 9, 10. Mai
1952, Zusammenfassung Nr. 4059d, Columbus,
Ohio, US; T.E. KING et al.: "Oxidative
dissimilation of nonnitrogenous compounds in
Acetobacter suboxydans", & SCIENCE 115, 14-
15(1952)

(73) Patentinhaber: BOEHRINGER MANNHEIM
GMBH
Sandhofer Strasse 116
D-6800 Mannheim 31 (DE)

(72) Erfinder: Ameyama, Minoru, Prof.
44-1, Ohuchi-Mihori
Yamaguchi 753 (JP)
Erfinder: Adachi, Osao, Prof.
2034 Miyanoshimi-Hirano
Yamaguchi 753 (JP)

(56) Entgegenhaltungen:
CHEMICAL ABSTRACTS, Band 51, Nr. 10, 10.
März 1957, Zusammenfassung Nr. 3729b-f,
Columbus, Ohio, US; A.C. ARCUS et al.: "Polyol
dehydrogenases. II. The polyol dehydrogenases
of Acetobacter suboxydans and Candida utilis",
& BIOCHEM. J. 64, 385-94(1956)

CHEMICAL ABSTRACTS, Band 54, Nr. 20, 25.
Oktober 1960, Zusammenfassung Nr. 21323g-i,
Columbus, Ohio, US; J. DE LEY et al.:
"Localization of oxidase systems in Acetobacter
cells", & BIOCHIM. ET BIOPHYS. ACTA 40,
277-89(1960)

Courier Press, Leamington Spa, England.

# EP 0 120 440 B1

(56) Entgegenhaltungen:
CHEMICAL ABSTRACTS, Band 99, Nr. 17, 24.
Oktober 1983, Seite 459, Zusammenfassung Nr.
138140w, Columbus, Ohio, US; S. OHMOMO et
al.: "Biotransformation of D-arabitol to D-
xylulose by Gluconobacter suboxydans
immobilized within K-carrageenan", & J.
FERMENT. TECHNOL. 1983, 61(4), 373-8

CHEMICAL ABSTRACTS, Band 92, Nr. 11, 17.
März 1980, Seite 454, Zusammenfassung Nr.
92684b, Columbus, Ohio, US; K. NABE et al.:
"Conversion of glycerol to dihydroxyacetone by
immobilized whole cells of Acetobacter
xylinum", & APPL. ENVIRON. MICROBIOL. 1979,
38(6), 1056-60

CHEMICAL ABSTRACTS, Band 91, Nr. 13, 24.
September 1979, Seite 472, Zusammenfassung
Nr. 106580h, Columbus, Ohio, US; S. YAMADA
et al.: "Studies on aerobic fermentation. V.
Enzymatic production of dihydroxyacetone by
Acetobacter suboxydans ATCC 621", & J.
FERMENT. TECHNOL. 1979, 57(3), 221-6
AGRIC. BIOL. CHEM., Band 49, Nr. 4, 1985,
Seiten 1001-1010; M. AMEYAMA et al.:
"Solubilization purification and properties of
membrane-bound glycerol dehydrogenase from
Gluconobacter industrius"

**Beschreibung**

Die Erfindung betrifft eine neue, NAD(P)-unabhängige Glycerindehydrogenase. Glycerindehydrogenasen katalysieren die Umsetzung von Glycerin unter Bildung von Dihydroxyaceton.

Die bisher bekannten Glycerindehydrogenasen sind Glycerin:-NAD(P)-Oxidoreduktasen (E.C.1.1.1.6, E.C.1.1.1.72 oder E.C.1.1.1.156), die NAD oder NADP in oxidierter Form als Coenzym benötigen. Die Umsetzung läßt sich durch das folgende Reaktionsschema wiedergeben:

$$(1) \qquad \text{Glycerin} + \text{NAD(P)}^+ \xrightleftharpoons[\text{dehydrogenase}]{\text{Glycerin-}} \text{Dihydroxyaceton} + \text{NAD(P)H} + \text{H}^+$$

Gleichung (1) stellt eine Gleichgewichtsreaktion dar, d.h. die NAD(P)-abhängigen Glycerindehydrogenasen katalysieren in gleicher Weise die Bildung von Glycerin aus Dihydroxyaceton. Soll daher Gleichung (1) zur Bestimmung von Glycerin bzw. der Glycerindehydrogenase-Aktivität benutzt werden, so ist es erforderlich, das Gleichgewicht durch Zugabe überschüssiger Mengen an NAD(P) auf die Seite des Dihydroxyacetons zu verschieben. Das führt dazu, daß diese Bestimmungsmethode relativ aufwendig und teuer ist. Geringe Glycerinmengen bzw. Glycerindehydrogenase-Aktivitäten lassen sich auf diesem Wege nicht exakt messen. Es ist ferner nachteilig, daß sich die Wasserstoffe nicht ohne weiteres vom NAD(P)H auf ein geeignetes Indikator-Redoxsystem übertragen lassen. Es ist hierzu ein weiteres Enzym erforderlich, beispielsweise Diaphorase, um die Wasserstoffe von NADH auf ein Tetrazoliumsalz unter Bildung von Formazan zu übertragen.

Aus Science Vol. 115, S. 14—15 (1952) ist bekannt, daß Acetobacter suboxydans in der Lage ist, Glycerin und Dihydroxyaceton zu oxidieren.

In Bioch. *64* S. 385—394 (1956) ist eine Dehydrogenase beschrieben, die in der Lage ist, Polyole zu oxidieren, wobei NAD nicht benötigt wird, und deren pH-Optimum bei pH 5.4 liegt.

Aufgabe der Erfindung war es, eine neue Glycerindehydrogenase zu finden, welche die genannten Nachteile nicht aufweist, mit deren Hilfe folglich Glycerin auf einfache und billige Weise bestimmt und auch geringe Mengen Glycerin möglichst quantitativ erfaßt werden können.

Gelöst wird diese Aufgabe durch Bereitstellen der erfindungsgemäßen NAD(P)-unabhängigen Glycerindehydrogenase. Das neue Enzym stellt eine neue Glycerindehydrogenase dar, welche die folgende Umsetzung katalysiert:

$$(2) \qquad \text{Glycerin} \xrightarrow[\text{dehydrogenase}]{\substack{\text{NAD(P)-unabhängige} \\ \text{Glycerin-}}} \text{Dihydroxyaceton} + 2\text{H}^+ + 2\text{e}$$

Die zwei Protonen und Elektronen werden dabei ohne Mitwirkung von NAD bzw. NADP als Coenzym auf geeignete Wasserstoff- und Elektronenakzeptoren übertragen. Da die Reaktion (2) vollständig in Richtung der Bildung von Dihydroxyaceton verläuft, ermöglicht es das neue Enzym, Glycerin und auch Triglyceride nach deren Verseifung zu Glycerin und freien Fettsäuren einfach und genau zu bestimmen.

Es ist auf diesem Wege möglich, auch sehr geringe Mengen von Glycerin und damit auch von Triglyceriden zu bestimmen. Die Mikrobestimmung von Glycerin bzw. von Triglyceriden mit Hilfe des erfindungsgemäßen Enzyms kann nicht nur nach der Endpunktmethode, sondern auch nach der kinetischen Methode erfolgen.

Die erfindungsgemäße NAD(P)-unabhängige Glycerindehydrogenase läßt sich durch die folgenden Eigenschaften näher charakterisieren:

**1. Wirkung**

Sie dehydriert Glycerin zu Dihydroxyaceton, wobei die abgespaltenen zwei Protonen und zwei Elektronen ohne Mitwirkung von NAD(P) als Coenzym auf geeignete Akzeptoren übertragen werden.

**2. Substratspezifität**

Sie reagiert in hohem Maß spezifisch mit Glycerin. Insbesondere gegenüber strukturell verwandten, üblicherweise ebenfalls in Körperflüssigkeiten vorkommenden Substanzen ist keine oder nur eine geringe Aktivität feststellbar. In der folgenden Tabelle werden die Aktivitäten gegenüber verschiedenen Substraten aufgeführt, wobei die Aktivität gegenüber Glycerin=100 gesetzt worden ist.

Tabelle

Relative Aktivität der erfindungsgemäßen Glycerindehydrogenase gegenüber verschiedenen Substraten (Glycerin=100)

| | |
|---|---|
| Glycerin | 100 |
| D-Sorbitol | 45 |
| D-Mannitol | 26 |
| D-Glucose | 0 |
| D-Fructose | 0 |
| Ethanol | 0 |

3. pH-Optimum und stabiler pH-Bereich

Der optimale pH-Bereich des erfindungsgemäßen Enzyms liegt im Bereich pH 7,0 bis 8,5. Die höchste Aktivität wird ungefähr bei pH 7,5 erreicht. Das Enzym ist in dem Bereich von pH 6,0 bis 10,0 stabil.

4. Temperaturoptimum der enzymatischen Wirkung

In dem Temperaturbereich von 25 bis 37°C weist das beanspruchte Enzym die höchste Aktivität auf.

5. Hemmung und Aktivierung

Oxamat wirkt leicht hemmend auf das Enzym. Phospholipide steigern die Aktivität der erfindungsgemäßen Glycerindehydrogenase um ca. 20%.

Die erfindungsgemäße Glycerindehydrogenase kann von einer ganzen Reihe von Mikroorganismen produziert werden, beispielsweise Pseudomonas-, Serratia-, Klebsiella-, Erwinia-, Aspergillus-, Penicillium-, Rhizopus-, Acetobacter- und Gluconobacter-Arten. In besonders guter Ausbeute wird das beanspruchte Enzym von Mikroorganismen der Gruppe Acetobacter und Gluconobacter produziert, beispielsweise Acetobacter xylinus DSM 2623 (IFO-3288), Gluconobacter cerinus DSM 2622 (IFO-3268) und Gluconobacter industrius DSM 2621 (IFO-3260). Die genannten Mikroorganismen-Stämme sind bei der Deutschen Sammlung für Mikroorganismen unter den angegebenen DSM-Nummern bzw. beim Institut für Fermentation, Osaka, unter den in Klammern angegebenen IFO-Nummern hinterlegt.

Um das beanspruchte Enzym zu produzieren, wird ein geeigneter Mikroorganismus nach bekannten Methoden in einem Kulturmedium kultiviert. Als Kulturmedium kann jedes übliche Kulturmedium benutzt werden, das allgemein als Nährstoffquelle für Bakterienkulturen bekannt ist.

Als Kohlenstoffquelle können insbesondere verschiedene metabolisierbare Kohlenstoffverbindungen benutzt werden, beispielsweise Glucose, Fructose, Succrose, Maltose, Saccharide mit 5 Kohlenstoffatomen, Melasse und organische Säuren (z.B. Gluconsäure und Essigsäure), Alkohole (z.B. Glycerin und Ethanol) und auch Zuckeralkohole (z.B. Mannit und Sorbitol).

Als Stickstoffquelle sind verschiedene Arten von Stickstoffverbindungen geeignet, beispielsweise Hefeextrakt, verdünntes Casein, corn steep liquor, Fleischextrakt (natürliche Matrialien), Harnstoff und Ammoniumsalze.

Als geeignete anorganische Verbindungen können dem Kulturmedium Natriumsalze, Kaliumsalze, Calciumsalze, Magnesiumsalze sowie verschiedene Phosphate zugesetzt werden.

Vorzugsweise wird die Kultivierung der Mikroorganismen zur Herstellung des erfindungsgemäßen Enzyms in flüssiger Phase durchgeführt. In technischem Maßstab sind jedoch submerse Kulturen besonders bevorzugt.

Die Bakterienkultur zu Züchtung des beanspruchten Enzyms wird vorzugsweise in dem Temperaturbereich von 28 bis 32°C gehalten. Die Temperatur kann jedoch auch geändert werden, so lange der Mikroorganismus wachsen und das gewünschte Enzym produzieren kann.

Die Kultivierungszeit hängt von den Kulturbedingungen ab. Üblicherweise wird eine maximale Enzymausbeute erhalten, wenn die Kultivierung nach ungefähr 1 bis 3 Tragen beendet wird.

Der pH-Wert der Kultivierungslösung ist nicht besonders kritisch. Er wird vorzugsweise in dem Bereich von pH 6 bis 7 eingestellt.

Die Aufarbeitung der Bakterienmasse mit dem gewünschten Emzym kann nach üblichen Methoden erfolgen. Die Bakterienmasse kann von flüssigen Medien, beispielsweise durch Niederdruckfiltration oder auch Zentrifugation getrennt werden. Zum Aufbrechen der Mikroorganismenzellen werden ebenfalls bekannte Methoden benutzt. Beispielsweise kann die gesammelte Bakterienmasse in einem geeigneten Puffer suspendiert werden und mit Hilfe von Ultraschall, einer French-Presse oder einer Dyno-Mühle aufgebrochen werden. Das erfindungsgemäße Enzym befindet sich in der Membranfraktion, die durch Hochgeschwindigkeitszentrifugation (90 Minuten bei 27.000 U/min) abgetrennt werden kann. Durch Zusatz eines geeigneten Detergens, gegebenenfalls in einem geeigneten Puffersystem, kann das Enzym aus der Membranfraktion herausgelöst werden.

Die erfindungsgemäße Glycerindehydrogenase wird aus der erhaltenen Lösung nach üblichen Verfahren zur Isolierung von Enzymen gewonnen. So läßt sich beispielsweise in vorteilhafter Weise das erfindungsgemäße Enzym in reiner Form durch Säulenchromatographie an Ionenaustauschern, wie Carboxymethyl- oder DEAE-Cellulose, durch Säulenchromatographie an Hydroxylapatit oder auch durch Gelfiltration an Sephadex isolieren.

Die Aktivität des erfindungsgemäßen Enzyms wird in internationalen Einheiten (U/mg) bei 25°C, pH 7,5, mit Glycerin, Phenazinmethosulfat und 2.6-Dichlorphenolindophenol als Substrat ausgedrückt.

Die erfindungsgemäße, NAD(P)-unabhängige Glycerindehydrogenase kann in vorteilhafter Weise zur Bestimmung von Glycerin gemäß Gleichung (2) verwendet werden. Nach dieser Reaktionsgleichung werden von einem Molekül Glycerin zwei Wasserstoffatome entfernt, um ein Molekül Dihydroxyaceton zu bilden. Die zwei Protonen und die zwei Elektronen werden auf geeignete Wasserstoffakzeptoren (A) übertragen. Es ergibt sich das folgende erweiterte Reaktionsschema:

$$\text{erfindungsgemäße}$$
$$\text{Glycerindehydrogenase}$$
$$(3) \qquad \text{Glycerin+A (oxidiert)} \xrightarrow{\hspace{5cm}} \text{Dihydroxyaceton+A-H}_2 \text{ (reduziert)}$$

Gemäß Reaktion (3) läßt sich sowohl Glycerin als auch die Glycerin-dehydrogenase-Aktivität bestimmen, indem entweder die Abnahme an dem oxidierten Akzeptor (A) oder die Zunahme an dem reduzierten Akzeptor (A) oder die Menge des gebildeten Dihydroxyacetons gemessen wird.

Gegenstand der Erfindung ist daher ferner ein Verfahren zur Bestimmung von Glycerin, dadurch gekennzeichnet, daß Glycerin in Gegenwart einer NAD(P)-unabhängigen Glycerindehydrogenase zu Dihydroxyaceton übergeführt wird, wobei zwei Protonen und zwei Elektronen direkt auf geeignete Akzeptoren übertragen werden und die Abnahme eines Akzeptors in der oxidierten Form bzw. die Zunahme eines Akzeptors in der reduzierten Form bzw. die Zunahme an Dihydroxyaceton gemessen wird.

Als Akzeptor (A) sind sämtliche Substanzen geeignet, die Elektronen und Protonen aufzunehmen vermögen. Soll der Akzeptor als Indikator für die enzymatische Reaktion herangezogen werden, so muß er beim Übergang von der oxidierten in die reduzierte Form eine meßbare Änderung aufweisen. Es sind solche Akzeptoren bevorzugt, die beim Übergang von der oxidierten in die reduzierte Form eine sichtbare Farbänderung zeigen, im Idealfalle von farblos zu gefärbt oder umgekehrt. Falls der Akzeptor bei dem Redox-Übergang keine meßbare Änderung zeigt, kann das erste Akzeptor-Redox-System mit einem zweiten Redox-System gekoppelt werden, das seinerseits einen meßbaren Redox-Prozeß aufweist.

Als Akzeptor im erfindungsgemäß brauchbaren Sinne kann beispielsweise Sauerstoff eingesetzt werden, der dabei in Wasser bzw. in andere reduzierte Sauerstofformen übergeht. Der Sauerstoffverbrauch kann in üblicher Weise, beispielsweise mit Hilfe einer Sauerstoffelektrode oder eines Warburg-Manometers gemessen werden. Die verbrauchte Menge an Sauerstoff ist der in der Probe vorhandenen Menge in Glycerin proportional.

Die Menge an in der Probe vorhandenem Glycerin läßt sich in bekannter Weise auch durch Messen des gebildeten Dihydroxyacetons bestimmen. Dem Fachmann stehen hier ebenfalls eine Reihe von bekannten Verfahren zur Verfügung (vgl. hierzu beispielsweise E. B. Sanders u. J. Schubert, Anal. Chem. *43* (1971) Seite 59 ff).

Besonders vorteilhaft ist die Verwendung von Akzeptoren, die bei dem Übergang von der oxidierten zu der reduzierten Form eine Farbänderung erfahren. Mit Hilfe spektrometrischer Methoden lassen sich solche Farbänderungen auf einfache und zuverlässige Weise messen.

Im folgenden werden einige Beispiele für erfindungsgemäß verwendbare Akzeptoren-Systeme aufgeführt, deren Redox-Vorgang direkt meßbar ist bzw. durch Kuppeln mit einem weiteren Redox-System meßbar gemacht werden kann:

1. Das $Fe^{2+}/Fe^{3+}$-System

Hierzu kann Kaliumhexacyanoferrat(III) eingesetzt werden, das nach Aufnahme eines Elektrons in das farblose Kaliumhexacyanoferrat(II) übergeht. Mit Eisen(III)-Ionen wird daraus der tief-blau gefärbte Komplex Berliner Blau gebildet.

$$(4) \qquad K_3[Fe(CN)_6] \xrightarrow{\;e\;} K_4[Fe(CN)_6] \xrightarrow{\;Fe3+\;} Fe_4[Fe(CN)_6]_3$$
$$\text{(schwach rot)} \qquad \text{(schwach gelb)} \qquad \text{Berliner Blau}$$

Es können auch direkt Eisen(III)-Salze eingesetzt werden. Die bei der Reduktion entstehenden Eisen(II)-Ionen können durch Komplexbildung mit geeigneten Farbstoffen, beispielsweise Bis-dimethylglyoxim oder auch 1,10-Phenanthrolin, nachgewiesen werden.

$$(5) \qquad Fe^{3+} \xrightarrow{\;e\;} Fe^{2+} \xrightarrow{\text{Bis-dimethyl-glyoxim}} Fe^{2+}\text{-bis-dimethylglyoxim (rot)}$$

$$(6) \qquad Fe^{3+} \xrightarrow{\;e\;} Fe^{2+} \xrightarrow{\text{1,10-Phen-anthrolin}} Fe^{2+}\text{-1,10-Phenanthrolin (Ferroin, rot)}$$

2. Di- oder Triarylmethanfarbstoffe

Das allgemeine Reaktionsschema für dieses Redox-System kann wie folgt dargestellt werden:

$$\text{oxidierte Form} \underset{\text{ox.}}{\overset{\text{red.}}{\rightleftarrows}} \text{reduzierte Form}$$
$$\text{(farbig)} \qquad \text{(Leukobase, farblos)}$$

Als Vertreter dieser Farbstoffklasse seien erwähnt: Malachitgrün, Kristallviolett, Coomassie-Blau bzw. deren Leukobasen.

3. Phenazinmethosulfat (PMS)

Allgemeines Schema:

$$(7) \qquad \text{PMS (ox.)} \xrightarrow{+2H^{\oplus}+2e} \text{PMS (red.)}$$

Der Übergang von der oxidierten zu der reduzierten Form von PMS ist nur schwer meßbar. Es ist von Vorteil, dieses Redox-System mit anderen Redox-Systemen zu koppeln. Bewährt hat sich die Kopplung mit Tetrazoliumsalzen. Das allgemeine Reaktionsschema läßt sich dann wie folgt wiedergeben:

$$(8) \qquad \text{PMS(red.)+Tetrazoliumsalz} \longrightarrow \text{PMS(ox.)+Formazan}$$
$$\text{(ox. Form, farblos)} \qquad \text{(red. Form, gefärbt)}$$

Als Tetrazoliumsalze können beispielsweise verwendet werden:

INT: 2-(p-Jodophenyl)-3-(p-nitrophenyl)-5-phenyl-tetrazolium-chlorid

MTT: 3-(4',5'-Dimethylthiazolyl-2-)-2,4-diphenyl-tetrazolium-bromid

NBT: 2,2'-Di(p-nitrophenyl)-5,5'-diphenyl-3,3'(3,3'-di-methoxy-4,4'-diphenylen)-ditetrazoliumchlorid

PMS läßt sich auch in vorteilhafter Weise mit 2.6-Dichlorphenolindophenol (DCIP) koppeln. Es gilt dann das folgende Redox-Schema:

$$(9) \qquad \text{PMS (red.)+DCIP (ox.)} \longrightarrow \text{PMS (ox.)+DCIP (red.)}$$

4. Indigo bzw. Indigo-Derivate

Indigo und seine Derivate wirken ebenfalls als Wasserstoffakzeptoren. Es gilt folgende allgemeine Schema:

$$(10) \qquad \text{Oxidierte Form} \xrightarrow{+2H} \text{reduzierte Form}$$
$$\text{(Blau)} \qquad \text{(farblos)}$$

Erfindungsgemäß verwendbare Indigo-Derivate sind beispielsweise 6,6'-Dibromindigo und 5,5',7,7'-Tetrabromindigo.

5. Phenoxazin- bzw. Phenothiazin-Farbstoffe

Auch Phenoxazin- bzw. Phenothiazin-Farbstoffe sind fähig, Wasserstoffatome aufzunehmen und gehen dabei von einer farbigen oxidierten Form in eine farblose reduzierte Form über.

$$(11) \qquad \text{Oxidierte Form} \xrightarrow{+2H} \text{reduzierte Form}$$
$$\text{(farbig)} \qquad \text{(farblos)}$$

Für diese Farbstoffklasse sei beispielhaft das Mehylenblau erwähnt, das durch Aufnahme von zwei Wasserstoffatomen pro Molekül in Leukomethylenblau übergeführt wird.

Die vorstehend beschriebenen Methoden zur Bestimmung von Glycerin lassen sich auch mit Vorteil für die Bestimmung von Substanzen, die in Glycerin übergeführt werden können, sowie für die Bestimmung von Enzymen, die solche Umsetzungen katalysieren, einsetzen.

Als Beispiel für die genannten Anwendungsmöglichkeiten wird die Bestimmung von Triglyceriden in Blut, Plasma oder Serum näher erläutert. Hierzu werden die Triglyceride (Neutralfette) mit Hilfe einer geeigneten Lipase in bekannter Weise hydrolysiert und das freigesetzte Glycerin mit Hilfe der erfindungsgemäßen Glycerindehydrogenase und geeigneten Elektronen- und Wasserstoffakzeptoren in der oben beschriebenen Weise umgesetzt. Die Reaktionsfolge läßt sich wie folgt darstellen:

$$(12) \qquad \text{Triglycerid} \xrightarrow{\text{Lipase}} \text{Glycerin+freie Fettsäuren}$$

$$\text{(3)} \qquad \text{Glycerin} + \text{A (oxidiert)} \xrightarrow[\text{Glycerindehydrogenase}]{\text{erfindungsgemäße}} \text{Dihydroxyaceton} + \text{A-H}_2 \text{ (reduziert)}$$

Die enzymatische Hydrolyse der Triglyceride wird nach bekannten Methoden durchgeführt. Als Enzyme zur Verseifung der Triglyceride können vorzugsweise handelsübliche Lipoproteinlipase (E.C.3.1.1.34) oder eine Mischung von Lipase (E.C. 3.1.1.3) und Esterase (E.C.3.1.1.1) benutzt werden.

Glucose im Blut beeinflußt die Bestimmung von Glycerin und von Triglyceriden mit Hilfe der erfindungsgemäßen, NAD(P)-unabhängigen Glycerindehydrogenase nicht.

Die Erfindung wird durch die folgenden Beispiele näher erläutert:

Beispiel 1

NAD(P)-unabhängige Glycerindehydrogenase aus Gluconobacter industrius DSM 2621 (IFO-3260)

Gluconobacter industrius DSM 2621 wird in einem Glycerin-Glutaminsäure-Medium nach bekannten Methoden kultiviert. Die Bakterienmasse wird gesammelt und in einer French-Presse bei einem Druck von 1000 kg/cm² aufgebrochen. Die nicht aufgebrochenen Zellen werden durch Zentrifugation (5000 g) entfernt. Das Zellhomogenat wird 90 Minuten in einer Ultrazentrifuge (68.000 g) zentrifugiert. Die Zellmembranfraktion wird gesammelt. Sie wird mit 0,05 M Tris-HCl-Puffer, pH 8,0, auf einen Gehalt von 10 mg Protein/ml eingestellt. Es wird ein übliches, geeignetes Detergens, beispielsweise Triton X 100, zugefügt, um eine Endkonzentration von 0,5% zu erlangen. Das erhaltene Gemisch wird 60 Minuten bei 0°C gerührt, um das Enzym in Lösung zu bringen. Anschließend wird 60 Minuten lang zentrifugiert (68.000 g). Aus dem Überstand wird nach Chromatographie an DEAE-Cellulose (pH 7,5) und Hydroxylapatit (pH 7,5) eine einheitliche Enzympräparation erhalten mit einer Aktivität von 50 U/mg Protein.

Beispiel 2

Anstelle des in Beispiel 1 genannten Mikroorganismus Gluconobacter industrius DSM 2621 wird Acetobacter xilinus DSM 2623 bzw. Gluconobacter cerinus DSM 2622 in der in Beispiel 1 angegebenen Weise kultiviert und aufgearbeitet. Es werden Enzympräparationen erhalten mit einer Aktivität von 42 U/mg bzw. 46 U/mg.

Beispiel 3

Bestimmung der Enzymaktivität der erfindungsgemäßen Glycerindehydrogenase

In einer Küvette werden 0,6 µmol Phenazinmethosulfat, 0,6 µmol 2.6-Dichlorophenolindophenol und 100 µmol Tris-HCl-Puffer, pH 7,5, vorgegeben. Es wird die zu bestimmende Enzympräparation, deren Aktivität in dem Bereich von 0,01 bis 0,1 Einheiten liegt, hinzugefügt. Durch Zugabe von zweifach destilliertem Wasser wird ein Endvolumen von 2,9 ml hergestellt. Nach Inkubation bei 25°C von 5 Minuten wird die Enzymreaktion durch Zugabe von 100 µmol Glycerin gestartet. Die Geschwindigkeit der Entfärbung von 2.6-Dichlorophenolindophenol wird durch Messen der Absorption bei 600 nm verfolgt. Die Berechnung der Enzymaktivität erfolgt, indem als millimolarer Absorptionskoeffizient für 2.6-Dichlorophenolindophenol bei pH 7,5 ein Wert von 100 angenommen wird. Die Enzymmenge, die eine Absorptionsänderung von $\Delta E_{600} = 2,33$/Min. bewirkt, wird als eine Einheit angenommen.

Beispiel 4

Bestimmung von Glycerin mit Phenazinmethosulfat und 2,6-Dichlorphenolindophenol 1. Erstellen einer Eichkurve

Aus einer Glycerinlösung (100 µmol/l) werden mit doppelt destilliertem Wasser sieben verschiedene Standardlösungen hergestellt, die 0,01, 0,02, 0,04, 0,06, 0,08, 0,10, 0,50 µmol Glycerin in 0,1 ml Lösung enthalten. Zu jeder dieser Glycerin-Standardlösungen wird 0,1 ml einer Enzymlösung mit einer Aktivität von 10 U zugegeben. Durch Zugabe von doppelt destilliertem Wasser wird ein Endvolumen von jeweils 2,8 ml eingestellt. Es wird 5 Minuten bei 25°C inkubiert.

Danach wird die Umsetzung gestartet durch Zugabe von 0,2 ml einer Lösung, die 0,6 µmol Phenazinmethosulfat und 0,6 µmol 2.6-Dichlorophenolindophenol enthält. Mit einem Spektrophotometer wird die Absorption bei 600 nm gemessen. Es wird ein linearer Verlauf in dem Bereich von 0,01 bis 0,05 µmol Glycerin beobachtet.

2. Messen einer unbekannten Probe

2 ml einer Probe mit unbekanntem Glyceringehalt werden in einer Küvette mit 2 ml einer Lösung versetzt, die 0,6 µmol Phenazinmethosulfat und 0,6 µmol 2.6-Dichlorophenolindophenol enthält. Es wird mit 0,5 ml Tris-HCl-Puffer, pH 7,5 (100 µmol) versetzt und 5 Minuten bei 25°C inkubiert. Die Nachweisreaktion wird gestartet durch Zugabe von 0,1 ml einer Enzymlösung mit einer Aktivität von 10 U. Die Abnahme der Absorption bei 600 nm wird gemessen. Durch Vergleich der gemessenen Absorptionsdifferenz mit der Eichkurve wird der Glyceringehalt der eingesetzten Probe bestimmt.

Beispiel 5

Bestimmung von Glycerin mit Phenazinmethosulfat und Tetrazoliumsalz

1 ml einer Probe mit unbekanntem Glyceringehalt werden in einer Küvette mit 2 ml einer Lösung versetzt, die 0,1 µmol Phenazinmethosulfat, 1,2 µmol 3 - (4,5 - Dimethylthiazolyl - 2) - 2,5 - diphenyl - tetrazoliumbromid und 100 µmol Tris-HCl-Puffer (0,1 M; pH 7,5) enthält. Es wird 5 Minuten bei 25°C inkubiert. Durch Zugabe von 20 µl einer Lösung mit 1 U des erfindungsgemäßen Enzyms wird die Nachweisreaktion gestartet. Die Absorptionsänderung bei 570 nm wird gemessen. Durch Vergleich der gemessenen Absorptionsdifferenz mit einer Eichkurve, die man durch Messen von Proben mit bekannten Glyceringehalten erstellen kann, wird der Glyceringehalt der eingesetzten Probe bestimmt.

Beispiel 6

Bestimmung von Triglyceriden

0,02 ml menschliches Serum werden in einer Küvette mit 0,5 ml einer Enzymlösung, die 100 bis 200 U Lipoproteinlipase oder ein Gemisch von Lipase und Esterase enthält, versetzt und 20 Minuten bei 25°C inkubiert.

- Danach werden 2 ml einer Lösung zugegeben, die 0,6 µmol Phenazinmethosulfat, 0,2 µmol 2.6-Dichlorophenolindophenol und 100 µmol Tris-HCl-Puffer, pH 7,5, enthält. Danach wird 5 Minuten bei 25°C inkubiert. Die Nachweisreaktion wird dann gestartet durch Zugabe von 0,1 ml einer Lösung, die 1 U der erfindungsgemäßen Glycerindehydrogenase enthält.

Die Absorptionsabnahme bei 600 nm wird gemessen. Aus der Absorptionsdifferenz kann in der in Beispiel 2 beschriebenen Weise der Glycerin-Gehalt und damit der Triglycerid-Gehalt ermittelt werden. Es wurde ein mittlerer Triglyceridgehalt von 110 mg/dl gefunden.

Bei der Bestimmung von Triglyceriden empfiehlt es sich, eine Leerwertbestimmung durchzuführen.

**Patentansprüche**

1. NAD(P)-unabhängige Glycerindehydrogenase.

2. Glycerindehydrogenase gemäß Anspruch 1, dadurch gekennzeichnet, daß sie Glycerin in Gegenwart eines Akzeptors in Dihydroxyaceton überführt.

3. Verfahren zur Gewinnung einer NAD(P)-unabhängigen Glycerindehydrogenase dadurch gekennzeichnet, daß man Acetobacter xylinus DSM 2623, Gluconobacter cerinus DSM 2622 oder Gluconobacter industrius DSM 2621 in einem Nährmedium züchtet und das Enzym aus der Kulturbrühe isoliert.

4. Verfahren zur Bestimmung von Glycerin, dadurch gekennzeichnet, daß Glycerin in Gegenwart einer NAD(P)-unabhängigen Glycerindehydrogenase gemäß Anspruch 1 in Gegenwart eines Akzeptors in Dihydroxyaceton übergeführt und die Abnahme eines Akzeptors in der oxidierten Form bzw. die Zunahme eines Akzeptors in der reduzierten Form bzw. die Zunahme an Dihydroxyaceton gemessen wird.

5. Verfahren gemäß Anspruch 4, dadurch gekennzeichnet, daß der als Akzeptor Sauerstoff verwendet und der Verbrauch an Sauerstoff gemessen wird.

6. Verfahren gemäß Anspruch 4, dadurch gekennzeichnet, daß das erste Akzeptor-Redoxsystem mit einem weiteren Redoxindikator gekoppelt wird.

7. Verfahren gemäß Anspruch 6, dadurch gekennzeichnet, daß als Akzeptor Phenazinmethosulfat und als weiterer Redoxindikator 2.6-Dichlorphenolindophenol verwendet und die Absorptionsabnahme von 2.6-Dichlorphenolindophenol bei 600 nm gemessen wird.

8. Verfahren gemäß Anspruch 6, dadurch gekennzeichnet, daß als Akzeptor Phenazinmethosulfat und als weiterer Redoxindikator ein Tetrazoliumsalz verwendet und die Formazanbildung photometrisch gemessen wird.

9. Verfahren zur Bestimmung von Triglyceriden durch Verseifen der Triglyceride zu Glycerin und freien Fettsäuren und Bestimmen des freien Glycerins gemäß einem der Ansprüche 4 bis 8.

10. Mittel zur Bestimmung von Glycerin sowie Triglyceriden, in dem gegebenenfalls Triglyceride zu Glycerin und freien Fettsäuren verseift werden, Glycerin mit Hilfe einer Glycerindehydrogenase in Gegenwart geeigneter Akzeptoren zu Dihydroxyaceton umgesetzt und die Abnahme oder Zunahme eines Akzeptors bzw. die Zunahme an Dihydroxyaceton gemessen wird, dadurch gekennzeichnet, daß es eine NADP-unabhängige Glycerindehydrogenase gemäß Anspruch 1 enthält.

11. NAD(P)-unabhängige Glycerindehydrogenase erhältlich aus Acetobacter xylinus DSM 2623, Gluconobacter cerinus DSM 2622 oder Gluconobacter industrius DSM 2621.

**Revendications**

1. Glycérol déshydrogénase NAD(P)-indépendante.

2. Glycérol déshydrogénase selon la revendication 1, caractérisée en ce qu'elle transforme le glycerol en dihydroxyacétone, en présence d'un accepteur.

3. Procédé pour l'obtention d'une glycérol déshydrogénase NAD(P)-indépendante, caractérisé en ce que l'on cultive l'Acetobacter xylinus DSM 2623, le Gluconobacter cerinus DSM 2622 ou le Gluconobacter industrius DSM 2621 dans un milieu nutritif et on isole l'enzyme à partir du bouillon de culture.

4. Procédé pour la détermination de glycérol, caractérisé en ce que l'on transforme le glycerol en dihydroxyacetone, en presence d'une glycerol déshydrogénase NAD(P)-indépendante selon la revendication 1, en présence d'un accepteur et que l'on mesure la diminution de l'accepteur sous la forme oxydée ou l'augmentation de l'accepteur sous la forme réduite ou l'augmentation en dihydroxyacetone.

5. Procédé selon la revendication 4, caractérisé en ce que comme accepteur, on utilise l'oxygène et que l'on mesure la consommation d'oxygène.

6. Procédé selon la revendication 4, caractérisé en ce que le premier systeme accepteur-redox est couplé avec un autre indicateur redox.

7. Procédé selon la revendication 6, caractérisé en ce que comme accepteur, on utilise le méthosulfate de phénazine et comme autre indicateur redox, le 2,6-dichlorophénolindophénol et que l'on mesure la diminution de l'absorption du 2,6-dichlorophénolindophénol à 600 nm.

8. Procédé selon la revendication 6, caractérisé en ce que comme accepteur, on utilise le méthosulfate de phénazine et comme autre indicateur redox, un sel de tétrazolium et que l'on mesure la formation de formazan par photométrie.

9. Procédé pour la détermination de triglycérides par saponification des triglycérides en glycérol et acides gras libres et détermination du glycérol libre selon l'une des revendications 4 à 8.

10. Agent pour la détermination de glycérol ainsi que de triglycérides, dans lequel éventuellement les triglycérides sont saponifiés en glycérol et acides gras libres, le glycérol est mis à reägir, à l'aide d'une glycéroldéshydrogénase, en présence d'un accepteur approprié, en dihydroxyacétone, et la diminution ou l'augmentation de l'accepteur ou l'augmentation de dihydroxyacétone est mesurée, caractérisé en ce qu'il contient une glycérol déshydrogénase NAD(P)-indépendante selon la revendication 1.

11. Glycérol déshydrogénase NAD(P)-indépendante pouvant être obtenue à partir de l'Acetobacter xylinus DSM 2623, du Gluconobacter cerinus DSM 2622 ou du Gluconobacter industrius, DSM 2621.

## Claims

1. NAD(P)-independent glycerol dehydrogenase.

2. Glycerol dehydrogenase according to claim 1, characterised in that it converts glycerol into dihydroxyacetone in the presence of an acceptor.

3. Process for the obtaining of an NAD(P)-independent glycerol dehydrogenase, characterised in that one cultures Acetobacter xylinus DSM 2623, Gluconobacter cerinus DSM 2622 or Gluconobacter industrius DSM 2621 in a nutrient medium and isolates the enzyme from the culture broth.

4. Process for the determination of glycerol, characterised in that glycerol is converted into dihydroxyacetone in the presence of an NAD(P)-independent glycerol dehydrogenase according to claim 1 in the presence of an acceptor and the decrease of an acceptor in the oxidised form or the increase of an acceptor in the reduced form or the increase of dihydroxyacetone is measured.

5. Process according to claim 4, characterised in that oxygen is used as acceptor and the consumption of oxygen is measured.

6. Process according to claim 4, characterised in that the first acceptor redox system is coupled with a further redox indicator.

7. Process according to claim 6, characterised in that phenazine methosulphate is used as acceptor and 2,6-dichlorophenolindophenol is used as further redox indicator and the absorption decrease of 3,6-dichlorophenolindophenol is measured at 600 nm.

8. Process according to claim 6, characterised in that phenazine methosulphate is used as acceptor and a tetrazolium salt as further redox indicator and the formazane formation is measured photometrically.

9. Process for the determination of triglycerides by saponification of the triglycerides to glycerol and free fatty acids and determination of the free glycerol according to one of claims 4 to 8.

10. Agent for the determination of glycerol, as well as of triglycerides, in which triglycerides are possibly saponified to glycerol and free fatty acids, glycerol is reacted to dihydroxyacetone with the help of a glycerol dehydrogenase in the presence of a suitable acceptor and the decrease or increase of an acceptor or the increase of dihydroxyacetone is measured, characterised in that it contains an NAD(P)-independent glycerol dehydrogenase according to claim 1.

11. NAD(P)-independent glycerol dehydrogenase obtainable from Acetobacter xylinus DSM 2623, Gluconobacter cerinus DSM 2622 or Gluconobacter industrius DSM 2621.